# EUROPEAN PATENT APPLICATION

(11) **EP 4 846 921 A2**
(43) Date of publication of application: **02.12.2026**
(21) Application number: 26205284.8
(22) Date of filing: 08.02.2019
(51) Int. Cl.: A61B 1/307

(54) **MEDICAL LASER APPARATUS AND SYSTEM**

(30) Priority: 09.02.2018 US 201862628513 P
(62) Document number(s) of the earlier application(s) in accordance with Art. 76 EPC: 24211085.6 / 4 516 203; 19750915.1 / 3 749 167
(71) Applicant: Gyrus ACMI, Inc. D.B.A. Olympus Surgical Technologies America, Westborough, MA 01581 (US)
(72) Inventor: Shelton, Kurt G., Bedford, MA 01730 (US); Chida, Masayasu, Tokyo, 192-8512 (JP)
(74) Representative: Noack, Andreas

(57) **Abstract**

A medical laser apparatus, including: an energy guide; a first energy source configured to generate energy for treating a target tissue through the energy guide; a second energy source configured to emit first and second aiming beams to a target tissue through the energy guide, the second aiming beam having at least one characteristic different from the first aiming beam; and a controller comprising hardware, the controller being configured to: receive a signal indicating an illumination mode from at least two illumination modes used by an endoscope to illuminate the target tissue; and control the second energy source to output the first or second aiming beam based on the indicated illumination mode.

## Description

### CROSS-REFERENCE TO RELATED APPLICATION

This application claims the benefit of U.S. Provisional Application No. 62/628,513 filed on February 9, 2018, the entire contents of which is incorporated herein by reference.

### BACKGROUND

### 1. Field

The invention relates generally to a medical laser apparatus and system and more particularly to a medical laser apparatus and system for use with an endoscope system having two or more illumination modes.

### 2. Prior Art

Medical lasers have been utilized in a variety of treatment procedures including, for example, various endoscopic procedures. Generally, these procedures require precisely controlled delivery of energy in order to successfully accomplish the desired procedure.

Generally, a surgical probe is utilized to deliver laser energy to a target tissue. The surgical probe generally comprises an energy guide, such as an optical fiber, coupled to an energy source, such as a laser, wherein the probe can be positioned such that the tip of the probe is positioned adjacent to the target tissue. Laser energy is directed out of the tip of the optical fiber onto desired portions of the target tissue. The laser optical fiber coupled to the laser source is required to be somewhat flexible such that the optical fiber can be manipulated. The laser system can include, for example, a Thulium Fiber Laser, which is used to generate the laser light for delivery through the optical fiber to the target tissue. The laser is capable of being operated in different treatment modes, such as a cutting (ablation) mode and a coagulation (hemostasis) mode.

The medical professional performing the particular procedure manipulates the optical fiber into position near the targeted tissue and sets the laser power and mode for various treatments, which may require different power and mode settings depending on the treatment, such as vaporization mode or coagulation mode.

The laser beam used for treating tissue is typically invisible to the human eye and to standard image sensors. Therefore, another illumination source can be used to generate a visible aiming beam. With the use of the aiming beam, an aiming beam spot can appear in the images formed when an endoscope is being used to view the target area.

Also, an endoscopic video imaging system has functions to assist the early detection of minute lesions, such as cancer and preoperative accurate diagnosis of diseased areas. The system incorporates specific light imaging functions using specific light spectra in addition to normal light imaging. The endoscopic video imaging system can have at least two illumination modes, white light (normal light) illumination and a specific light illumination mode. The endoscope also has an illumination mode switching function that changes from the white light mode to specific light illumination mode or from specific light illumination mode to the white light mode.

### SUMMARY

Accordingly, a medical laser apparatus is provided. The medical laser apparatus comprising: an energy guide; a first energy source configured to generate energy for treating a target tissue through the energy guide; a second energy source configured to emit first and second aiming beams to a target tissue through the energy guide, the second aiming beam having at least one characteristic different from the first aiming beam; and a controller comprising hardware, the controller being configured to: receive a signal indicating an illumination mode from at least two illumination modes used by an endoscope to illuminate the target tissue; and control the second energy source to output the first or second aiming beam based on the indicated illumination mode.

Wherein when a white light illumination mode is indicated, the controller can control the second energy source to emit the first aiming beam having a wavelength in the range of 500 nm to 550 nm.

Wherein when a special light illumination mode is indicated, the controller can control the second energy source to emit the first aiming beam having a wavelength in the range of 635 nm to 690 nm. The special light mode can be one of a narrow band imaging mode, an auto fluorescence imaging mode or an infrared imaging mode.

The controller can be further configured to receive a signal indicating whether a spot caused by the first or second aiming beam can be identified in an image from the endoscope. When the spot cannot be identified in the image, the controller can be further configured to switch one of the first or second aiming beams to an other of the first or second aiming beams. The controller can be further configured to receive a signal indicating whether a spot caused by the other of the first or second aiming beam can be identified in the image from the endoscope. When the spot from the other of the first or second aiming beam cannot be identified in the image, the controller can be configured to control the first energy source to prohibit the first energy source from generating energy for treating the target tissue.

The at least one characteristic can be selected from a group consisting of wavelength, power level and emitting pattern.

The energy guide can be a laser fiber.

The first energy source can be a treatment laser beam.

Also provided is an endoscope controller comprising hardware, where the endoscope controller is for use with an endoscope. The endoscope controller being configured to: output a first signal indicating an illumination mode of the endoscope; detect whether a spot from an aiming beam generated by an aiming beam energy source is visible in an image captured by an image sensor in the endoscope; and outputting a second signal based on the detection.

The second signal can be output only where the spot cannot be detected in the image.

The aiming beam can be a first aiming beam; and where the spot cannot be detected in the image, the second signal can instruct a laser apparatus to one of change the first aiming beam to a second aiming beam having at least one characteristic different from the first aiming beam.

Still further provided is a medical system comprising: a medical laser apparatus, comprising: an energy guide; a first energy source configured to generate energy for treating a target tissue through the energy guide; a second energy source configured to emit first and second aiming beams to a target tissue through the energy guide, the second aiming beam having at least one characteristic different from the first aiming beam; and a first controller comprising hardware, the first controller being configured to: receive a first signal indicating an illumination mode from at least two illumination modes used by an endoscope to illuminate the target tissue; control the second energy source to output the first or second aiming beam based on the indicated illumination mode; and a second controller comprising hardware, the second controller being for use with an endoscope, the second controller being configured to: output the first signal to the first controller indicating the illumination mode from the at least two illumination modes used by the endoscope.

When a white light illumination mode is indicated, the first controller can control the second energy source to emit the first aiming beam having a wavelength in the range of 500 nm to 550 nm.

When a special light illumination mode is indicated, the first controller can control the second energy source to emit the first aiming beam having a wavelength in the range of 635 nm to 690 nm. The special light mode can be one of a narrow band imaging mode, an auto fluorescence imaging mode or an infrared imaging mode.

The second controller can be further configured to: output a second signal indicating whether a spot caused by the first or second aiming beam can be identified in an image from the endoscope; and the first controller can be further configured to: receive the second signal; and when the spot cannot be identified in the image, switch one of the first or second aiming beams to an other of the first or second aiming beams.

The second controller can be further configured to: output a second signal indicating whether a spot caused by the first or second aiming beam can be identified in an image from the endoscope; and the first controller can be further configured to: receive the second signal; and when the spot from the other of the first or second aiming beam cannot be identified in the image, control the first energy source to prohibit the first energy source from generating energy for treating the target tissue.

The at least one characteristic can be selected from a group consisting of wavelength, power level and emitting pattern.

The energy guide can be a laser fiber.

The first energy source can be a treatment laser beam.

### BRIEF DESCRIPTION OF THE DRAWINGS

These and other features, aspects, and advantages of the present invention will become better understood when the following detailed description is read with reference to the accompanying drawings in which like characters represent like parts throughout the drawings, wherein:
FIG. 1 illustrates a medical system having an endoscope, laser apparatus, endoscope processor and endoscope light source.
FIG. 2 illustrates a schematic view of the medical system of FIG. 1 including a distal-end of the endoscope of FIG. 1.
FIG. 3 illustrates a flow chart of a first method of operation of the medical system of FIG. 1.
FIG. 4 illustrates a flow chart of a second method of operation of the medical system of FIG. 1.

### DETAILED DESCRIPTION

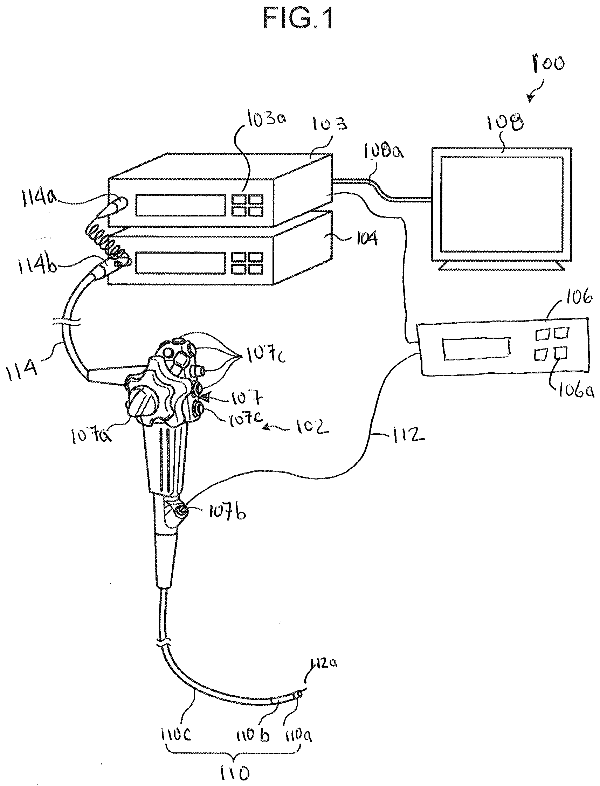

Referring now to FIG. 1, the same illustrates an overall configuration of a medical system 100 having an endoscope 102, an endoscope processor 103, a light source 104, a laser apparatus 106 and a display 108. As illustrated in FIG. 1, the endoscope 102 includes an insertion section 110 configured to be inserted into a subject that images the inside of the subject and generates an image signal of the inside of the subject, the endoscope processor 103 that performs predetermined image processing on the image signal captured by the endoscope 102 and controls at least parts of medical system 100, the light source 104 that generates illumination light of the endoscope 102 having at least two illumination modes, the laser apparatus 106 having a first energy source for generating energy for treating a target tissue through an energy guide 112 and a second energy source configured to emit two or more aiming beams to a target tissue through the energy guide 112, and the display device 108 that displays the aiming beam and an image of the image signal having been subject to the image processing performed by the endoscope processor 103.

The endoscope 102 includes the insertion portion 110 to be inserted into the subject, an operating unit 107 to be held by an operator, which is on a proximal end portion side of the insertion portion 110, and a flexible universal cord 114 extended from the operating unit 107. Although FIG. 1 illustrates a Gastrointestinal (GI) endoscope, the apparatus and systems disclosed herein are not limited to a GI endoscope and also have particular utility for use with other types of endoscopes, such as ureteroscope or cystoscope or those used for a other treatment procedures.

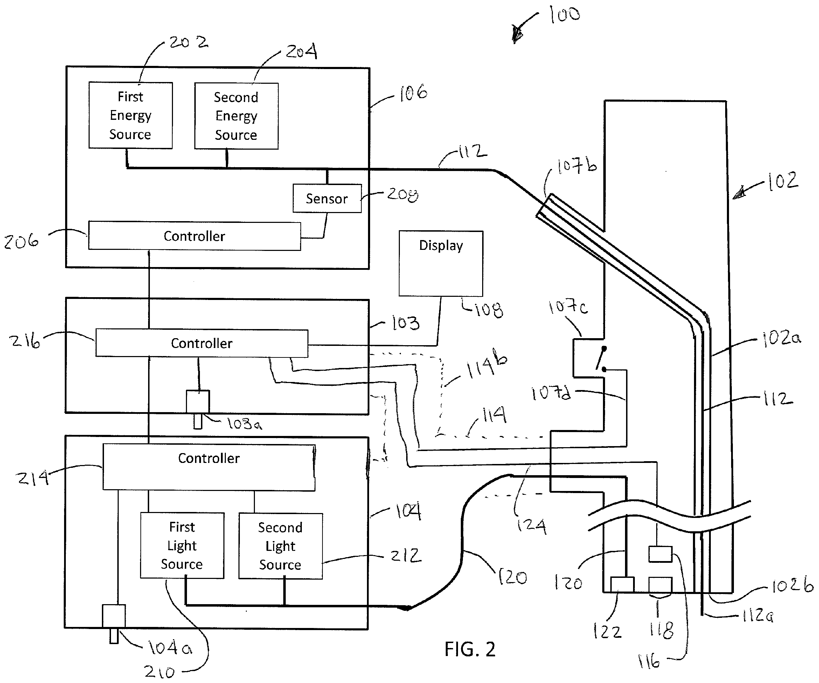

The insertion portion 110 is formed using a lighting fiber (light guide), an electric cable, an optical fiber, and the like. The insertion portion 110 includes a distal end portion 110a incorporating an imaging unit to be described later, a bendable bend portion 110b including a plurality of bend pieces, and a flexible tube portion 110c provided on a proximal end portion side of the bend portion 110b, which is flexible. The distal end portion 110a is provided with an illumination light guide 120 (see FIG. 2) that illuminates the inside of the subject via an illumination lens 122 (see FIG 2), an observation unit, including an image sensor, such as a CCD or CMOS and an objective lens system 118 (see FIG. 2) that images the inside of the subject, an insertion port 107b that communicates with a treatment tool channel 102a (see FIG. 2), and an air/water supply nozzle (not illustrated).

The operating unit 107 includes a bending knob 107a for bending the bend portion 110b in the up and down direction and the right and left direction, the treatment tool insertion port 107b through which a treatment tool, such as medical forceps or the energy guide 112 is inserted into a body cavity of the subject, and a plurality of switches 107c for operating a peripheral device such as the endoscope processor 103, the light source device 104, an air supply device, a water supply device, and a gas supply device. The treatment tool, such as the energy guide 112 can be inserted from the treatment tool insertion port 107b and through the channel 102a such that a distal end thereof is exposed from an opening 102b (see FIG. 2) of the channel 102a at the distal end of the insertion portion 110.

The universal cord 114 includes a lighting fiber, a cable, and the like. The universal cord 114 is branched at the proximal end thereof. One end of the branched ends is a connector 114a, and the other proximal end of the branched ends is a connector 114b. The connector 114a is attachable/detachable to/from a connector of the endoscope processor 103. The connector 114b is attachable/detachable to/from the light source 104. The universal cord 114 propagates the illumination light emitted from the light source 104 to the distal end portion 110a via the connector 114b and the light guide 120 (see FIG. 2). Further, the universal cord 114 transmits an image signal captured by the image sensor 116 (see FIG. 2) to be described later to the endoscope processor 103 via a signal line 124 (see FIG. 2) in the cable and via the connector 114a.

The endoscope processor 103 executes predetermined image processing on the image signal output from the connector 114a, and controls at least part of the components making up the medical system 100.

The light source 104 includes one or more light sources that emit light having one or more illumination characteristics, referred to as illumination modes, a condenser lens, and the like. Such light sources can be, for example, a Xenon lamp, an LED (Light-Emitting Diode), an LD (Laser Diode), or any combination thereof. Under the control of the endoscope processor 103, the light source 104 emits light from the one or more light sources thereof, and supplies the light to the endoscope 102 connected via the connector 114b and the light guide of the universal cord 114 as illumination light for the inside of the subject as an object. The illumination modes can be a white light illumination mode or a special light illumination mode, such as a narrow band imaging mode, an auto fluorescence imaging mode or an infrared imaging mode. A special light illumination can concentrate and intensify specific wavelengths of light, for example, resulting in a better visualization of a superficial micro-vessel and mucosal surface structures to enhance the subtle contrast of the irregularities of the mucosa.

The display 108 includes, for example, a liquid crystal display, an organic electro luminescence (EL) display, or the like. The display 108 displays various kinds of information including the image having been subject to predetermined image processing by the information processing apparatus 103 via a video cable 108a. This allows an operator to observe and determine behavior of the desired position inside the subject by operating the endoscope 102 while watching the image (in-vivo image) displayed by the display 108.

Referring now to Figure 2, the medical system 100 of FIG. 1 is shown schematically. The laser apparatus 106 is for use with the energy guide 112, such as a laser fiber. The energy guide is disposed in the channel 102a through the treatment insertion port 107b and includes a distal end 112a that extends distally from the distal end opening 102b of the channel 102a so as to be configured to direct treatment energy to the target tissue. A proximal end of the energy guide 112 is operatively connected to the laser apparatus 106.

The laser apparatus 106 includes two or more energy sources for generating laser energy coupled to the proximal end of the energy guide 112. Such energy sources can be selectable by a user by an input, such as a button 106a on the laser apparatus 106 or a foot switch (not shown), through software or a user interface on the display 108 or other inputs, manual or automatic as are known in the art. A first energy source 202 is optically coupled to the energy guide 112 and can be configured to generate energy for treating the target tissue through the energy guide 112. For example, the first energy source 202 can be a thulium laser, used to generate laser light for delivery through the light guide 112 to the target tissue to operate in different treatment modes, such as a cutting (ablation) mode and a coagulation (hemostasis) mode. Other energy sources known in the art for such treatment of tissue, or any other treatment modes, can also be used for the first energy source 202, such as Ho:YAG, Nd:YAG and CO₂ as well as others known in the art.

The two or more energy sources can also include a second energy source 204 also optically coupled to the energy guide 112 and configured to emit at least two aiming beams to the target tissue through the energy guide 112, where the first aiming beam has at least one characteristic different from the second aiming beam. Such differing characteristics can be wavelength, power level and/or emitting pattern. For example, the first aiming beam can have a wavelength in the range of 500 nm to 550 nm while the second aiming beam can have a wavelength in the range of 635 nm to 690 nm. The characteristics of the different aiming beams can be selected based on the visibility of the aiming beams in the image processed by the endoscope processor 103 and displayed on the display 108 under certain illumination modes provided by the light source 104.

The laser apparatus 106 further includes a controller 206 comprising hardware, such as a CPU, that controls the operation of the laser apparatus 106 including the first and second energy sources 202, 204. The laser apparatus 106 can further include a sensor 208 operatively coupled to the energy guide 112 and under the control of the controller 206. The sensor 208 is configured, as known in the art, to detect reflected light through the energy guide 112 from the distal end 112a of the energy guide 112 and back to the sensor 208 such that the sensor 208 can determine an illumination mode output to the light guide 120 from the light source 104. That is, such sensor 208 detects the illumination mode being used to illuminate the target tissue. Such reflected light detection can be similar to that described in United States Patent 5,860,972 issued on January 19, 1999, the contents of which is incorporated herein by reference.

The light source 104 includes one or more light sources, such as a first light source 210 and a second light source 212 under the control of a controller 214. The light sources 210, 212 can be selected by a user through an input, such as a button 104a on the light source 104 or a foot switch (not shown), through software or a user interface on the display 108 or other inputs, manual or automatic as are known in the art. The first and second light sources 210, 212 are optically coupled to the light guide 120 to provide different illumination modes, as described above, to the light guide 120. Although a different light source is shown for each illumination mode, a single light source can be provided to produce illumination modes having different characteristics through the use of filters, lens and the like.

The endoscope processor 103 also includes a controller 216 comprising hardware, such as a CPU, for control of the endoscope 102, display 108, light source 104 and/or laser apparatus 106. The controller 216, as discussed above, receives a signal from the image sensor 116 through line 124 in the universal cord 114 to process the same so as to generate an image/video for viewing on the display 108. Such image includes not only the target area of the tissue to be treated under the illumination of the light source 104 but also an aiming beam generated by the laser apparatus 106 when the first energy source 202 is active and the energy guide 112 is being used to treat the target tissue. The endoscope processor 103 includes one or more inputs, such as a button 103a on the endoscope processor 103 or a foot switch (not shown), through software or a user interface on the display 108 or other inputs, manual or automatic as are known in the art.

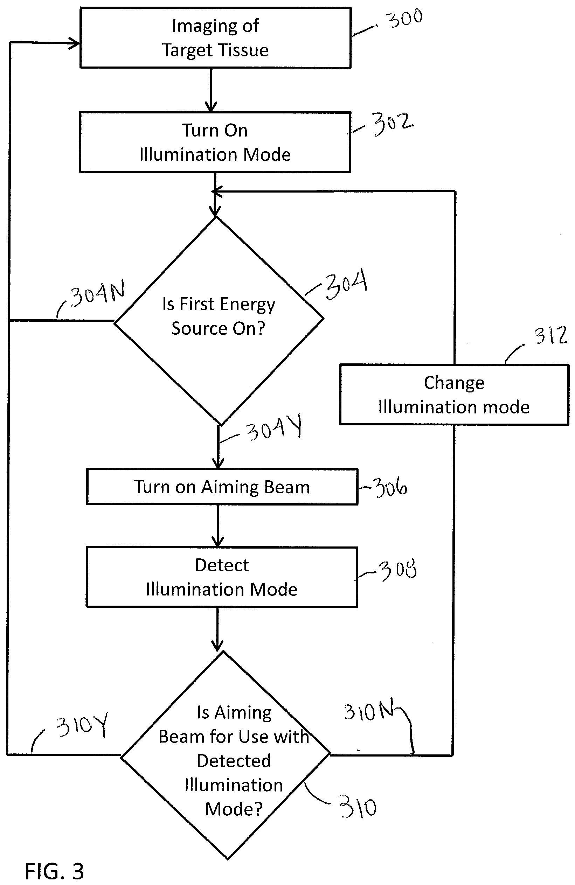

A use of the medical system 100 of FIG. 1 will now be described with regard to the flow chart illustrated in FIG. 3. After insertion of the endoscope 102 to the target tissue site, the user views the target tissue on the display 108 at 300. Such viewing of the target tissue is with an illumination mode set at 302 and output by one of the light sources 210, 212 of the light source 104. Such illumination mode can be set by selection by the user by any means known in the art or automatically provided by a determination made by either of controllers 214, 216 based on predetermined criteria.

A determination is made at 304 by the controller 206 as to whether the first energy source 202 is activated (on) and delivering treatment energy to the energy guide 112. Where it is determined that the first energy source 202 is not delivering treatment energy to the energy guide 112, the controller 206, at 304N, does not activate the second energy source 204 to generate an aiming beam. Where it is determined that the first energy source 202 is delivering treatment energy to the energy guide 112, the controller 206, at 304Y, activates the second energy source 204, at 306, to generate one of the first or second aiming beams.

At 308, the controller 206 determines the illumination mode from the illumination modes used by the endoscope to illuminate the target tissue, such as receiving a signal indicating the type of illumination mode being used. The illumination mode signal provided to the controller 206 can be a manual input from the user at input 104a of the light source 104 to direct the light source controller 214 to output a signal to the controller 216 of the endoscope processor 103, which in turn outputs a signal to the laser apparatus controller 206. Such manual input can also be from the input, such as button 103a, of the endoscope processor 103. The light source controller 214 can also directly output a signal indicating the illumination mode to the laser apparatus controller 206. The input can also be via a button 107c on the endoscope through signal line 107d to the controller 216 of the endoscope processor 103, which is in turn relayed to the controller 206 of the laser apparatus. The controller 206 of the laser apparatus 106 can also receive a signal indicative of the illumination mode used by the endoscope from the sensor 208, which detects the illumination being used by reflected light through the energy guide 112 and the controller 206 processes such detection and determines the illumination mode based on the output from the sensor 208. Furthermore, the controller 216 of the endoscope processor 103 can analyze the image signal from the image sensor 116 and determine an illumination mode based on such image signal and output such determination to the controller 206 of the laser apparatus 106. Other sensors (not shown) may also be employed for determination of the illumination mode being used by the endoscope, such as at the distal end of the endoscope 102 or in the endoscope processor 103.

At 310, a determination is made as to whether the aiming beam is appropriate for the determined illumination mode. Such determination can be based on historical data reflected in a look up table (LUT) operatively connected to the controller 206, where the LUT corresponds illumination mode to aiming beam characteristic. Such LUT can store data of illumination modes (or the wavelength of the illumination light) and a corresponding wavelength of aiming beam for use with such illumination mode or wavelength of such illumination mode. Where it is determined that the aiming beam being used is appropriate for use with the illumination mode being used, no change is required in the aiming beam being used and the process continues at 310Y to image the target tissue until the determination is made at 310N that the aiming beam being used is not appropriate for use with the illumination mode being used. Such determination can be made upon predetermined intervals or upon an occurrence of a predetermined event, such as the first energy source being turned off and then again on.

However, where it is determined, at 310N, that the aiming beam being used is not appropriate for use with the illumination mode being used, the second energy source 204 is controlled to change the aiming beam at 312 based on the indicated illumination mode. For example, when a white light illumination mode is determined, the controller 206 can control the second energy source 204 to emit a first aiming beam having a wavelength in the range of 500 nm to 550 nm. Alternatively, where a special light illumination mode is determined, the controller 206 can control the second energy source 204 to emit the second aiming beam having a wavelength in the range of 635 nm to 690 nm. As discussed above, the special light mode can be, for example, one of a narrow band imaging mode, an auto fluorescence imaging mode or an infrared imaging mode.

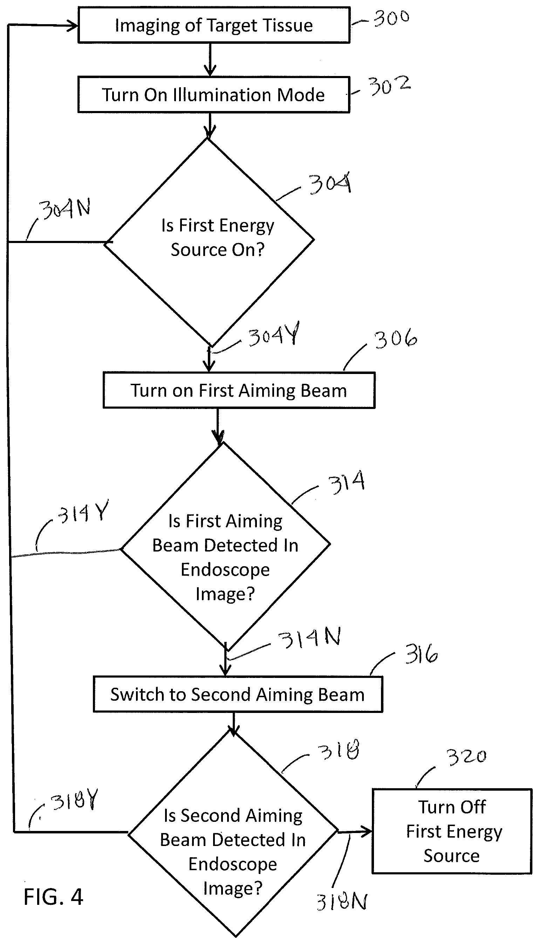

Another use of the medical system 100 of FIG. 1 will now be described with regard to the flow chart illustrated in FIG. 4, in which 300, 302, 304 and 306 are substantially as described above. Where one of the aiming beams, such as the first or second aiming beams is activated by the laser apparatus 106, a determination is made at 314 as to whether a spot caused by the first or second aiming beam can be identified in an image from the endoscope 102. That is, the controller 216 analyzes the image signal from the image sensor 116 to determine if the aiming beam spot being used is visible in the image of the target tissue. Such determination of a spot in image data is well known in the art, such as by pixel comparison to determine a disparity (a discontinuity) in pixel data in an area of the image corresponding to an expected size and/or shape of the spot. Furthermore, such determination may consider the spot to not be visible if a spot is detected but the disparity is below some predetermined threshold where a user would have trouble identifying the spot clearly from the image.

Where the spot is detected, or is sufficiently detected in the image, the imaging, illumination and display of the image and spot continues, at 314Y, until such determination changes or the laser apparatus no longer activates the first energy source 202. Where the spot cannot be detected or not sufficiently detected in the image at 314N, the controller 216 outputs a signal, at 316, to the laser apparatus controller 206 to switch to a different one of the first or second aiming beams.

A similar determination is made by the controller 216 at 318 to determine whether a spot caused by the different one of the first or second aiming beams can be identified in the image from the endoscope 102. Where the spot is detected, or is sufficiently detected in the image at 318Y, the imaging, illumination and display of the image and spot continues until such determination changes or the laser apparatus no longer activates the first energy source 202. Where the spot cannot be detected or not sufficiently detected in the image at 318N, as a safety measure, the controller can output a signal to the laser apparatus controller 206 to control the first energy source to prohibit the first energy source from generating energy for treating the target tissue at 320.

Although described with regard to a flexible endoscope, the above apparatus and methods also have utility for rigid type endoscopes. In addition, although the laser apparatus 106 is described as a separate device, the features thereof can be incorporated into one or both of the light source and endoscope processor, in which a common controller can be used to make the determinations and control indicated herein.

While there has been shown and described what is considered to be preferred embodiments, it will, of course, be understood that various modifications and changes in form or detail could readily be made without departing from the spirit of the invention. It is therefore intended that the invention be not limited to the exact forms described and illustrated, but should be constructed to cover all modifications that may fall within the scope of the appended claims.

Hereinafter, some further examples are provided:
Example 1. A medical laser apparatus, comprising:
   an energy guide;
   a first energy source configured to generate energy for treating a target tissue through the energy guide;
   a second energy source configured to emit first and second aiming beams to a target tissue through the energy guide, the second aiming beam having at least one characteristic different from the first aiming beam; and
   a controller comprising hardware, the controller being configured to:
      receive a signal indicating an illumination mode from at least two illumination modes used by an endoscope to illuminate the target tissue; and
      control the second energy source to output the first or second aiming beam based on the indicated illumination mode.
Example 2. The medical laser apparatus according to Example 1, wherein when a white light illumination mode is indicated, the controller controls the second energy source to emit the first aiming beam having a wavelength in the range of 500 nm to 550 nm.
Example 3. The medical laser apparatus according to Example 1, wherein when a special light illumination mode is indicated, the controller controls the second energy source to emit the second aiming beam having a wavelength in the range of 635 nm to 690 nm.
Example 4. The medical laser apparatus according to Example 3, wherein the special light mode is one of a narrow band imaging mode, an auto fluorescence imaging mode or an infrared imaging mode.
Example 5. The medical laser apparatus according to Example 1, wherein the controller is further configured to receive a signal indicating whether a spot caused by the first or second aiming beam can be identified in an image from the endoscope.
Example 6. The medical laser apparatus according to Example 5, wherein, when the spot cannot be identified in the image, the controller being further configured to switch one of the first or second aiming beams to an other of the first or second aiming beams.
Example 7. The medical laser apparatus according to Example 6, wherein the controller is further configured to receive a signal indicating whether a spot caused by the other of the first or second aiming beam can be identified in the image from the endoscope.
Example 8. The medical laser apparatus according to Example 7, wherein, when the spot from the other of the first or second aiming beam cannot be identified in the image, the controller is configured to control the first energy source to prohibit the first energy source from generating energy for treating the target tissue.
Example 9. The medical laser apparatus according to Example 1, wherein the at least one characteristic is selected from a group consisting of wavelength, power level and emitting pattern.
Example 10. The medical laser apparatus according to Example 1, wherein the energy guide is a laser fiber.
Example 11. The medical laser apparatus according to Example 1, wherein the first energy source is a treatment laser beam.
Example 12. An endoscope controller comprising hardware, the endoscope controller being for use with an endoscope, the endoscope controller being configured to:
   output a first signal indicating an illumination mode of the endoscope;
   detect whether a spot from an aiming beam generated by an aiming beam energy source is visible in an image captured by an image sensor in the endoscope; and
   outputting a second signal based on the detection.
Example 13. The endoscope controller of Example 12, wherein the second signal is output only where the spot cannot be detected in the image.
Example 14. The endoscope controller of Example 12, wherein:
   the aiming beam is a first aiming beam; and
   where the spot cannot be detected in the image, the second signal instructs a laser apparatus to one of change the first aiming beam to a second aiming beam having at least one characteristic different from the first aiming beam.
Example 15. A medical system comprising:
   a medical laser apparatus, comprising:
      an energy guide;
      a first energy source configured to generate energy for treating a target tissue through the energy guide;
      a second energy source configured to emit first and second aiming beams to a target tissue through the energy guide, the second aiming beam having at least one characteristic different from the first aiming beam; and
      a first controller comprising hardware, the first controller being configured to:
         receive a first signal indicating an illumination mode from at least two illumination modes used by an endoscope to illuminate the target tissue;
         control the second energy source to output the first or second aiming beam based on the indicated illumination mode; and
   a second controller comprising hardware, the second controller being for use with an endoscope, the second controller being configured to:
      output the first signal to the first controller indicating the illumination mode from the at least two illumination modes used by the endoscope.
Example 16. The medical system of Example 15, wherein when a white light illumination mode is indicated, the first controller controls the second energy source to emit the first aiming beam having a wavelength in the range of 500 nm to 550 nm.
Example 17. The medical system according to Example 15, wherein when a special light illumination mode is indicated, the first controller controls the second energy source to emit the first aiming beam having a wavelength in the range of 635 nm to 690 nm.
Example 18. The medical system according to Example 17, wherein the special light mode is one of a narrow band imaging mode, an auto fluorescence imaging mode or an infrared imaging mode.
Example 19. The medical system according to Example 15, wherein:
   the second controller is further configured to:
      output a second signal indicating whether a spot caused by the first or second aiming beam can be identified in an image from the endoscope; and
   the first controller is further configured to:
      receive the second signal; and
      when the spot cannot be identified in the image, switch one of the first or second aiming beams to an other of the first or second aiming beams.
Example 20. The medical system according to Example 15, wherein:
   the second controller is further configured to:
      output a second signal indicating whether a spot caused by the first or second aiming beam can be identified in an image from the endoscope; and
   the first controller is further configured to:
      receive the second signal; and
      when the spot from the other of the first or second aiming beam cannot be identified in the image, control the first energy source to prohibit the first energy source from generating energy for treating the target tissue.
Example 21. The medical system according to Example 15, wherein the at least one characteristic is selected from a group consisting of wavelength, power level and emitting pattern.
Example 22. The medical system according to Example 15, wherein the energy guide is a laser fiber.
Example 23. The medical system according to Example 15, wherein the first energy source is a treatment laser beam.

## Claims

1. A medical system for providing treatment to a target in a patient, the medical system comprising:
at least one laser apparatus comprising a first energy source configured to generate treatment energy for the target and a second energy source configured to emit an aiming beam toward the target;
a light source, external to the at least one laser apparatus, configured to illuminate the target; and
a controller circuit configured to:
receive an image of the illuminated target while the aiming beam is emitted toward the target;
determine, based on the image, whether a spot produced by the aiming beam is identifiable in the image; and
control operation of the at least one laser apparatus based at least in part on the determination, including preventing the first energy source from generating the treatment energy or directing the treatment energy to the target in response to determining that the spot is not identifiable in the image.

2. The medical system of claim 1, further comprising an imaging sensor, wherein the light source has at least two selectable illumination modes for illuminating the target,
wherein the controller circuit is further configured to receive an indication of a selected one of the at least two selectable illumination modes and control the second energy source to emit a first aiming beam or a second aiming beam based at least in part on the indicated illumination mode, the second aiming beam having at least one characteristic different from the first aiming beam.

3. The medical system of claim 2, comprising an endoscope including the imaging sensor, wherein the endoscope is operably coupled to the light source and the controller circuit.

4. The medical system of claim 2, wherein the controller circuit is configured to control the second energy source to emit the first aiming beam when a white-light illumination mode is indicated, the first aiming beam having a wavelength in a range from 500 nm to 550 nm.

5. The medical system of claim 2, wherein the controller circuit is configured to control the second energy source to emit the second aiming beam when a special-light illumination mode is indicated, the second aiming beam having a wavelength in a range from 635 nm to 690 nm.

6. The medical system of claim 5, wherein the special-light illumination mode comprises at least one of a narrow-band imaging mode, an autofluorescence imaging mode, or an infrared imaging mode.

7. The medical system of claim 2, wherein the controller circuit is configured to control the second energy source to switch from the first aiming beam to the second aiming beam in response to determining that a spot produced by the first aiming beam is not identifiable in an image received from the imaging sensor.

8. The medical system of claim 7, wherein the controller circuit is configured to prevent the first energy source from generating the treatment energy in response to determining that a spot produced by the second aiming beam is not identifiable in a subsequent image received from the imaging sensor.

9. The medical system of claim 2, wherein the at least one characteristic comprises at least one of wavelength, power level, or emitting pattern.

10. The medical system of claim 1, wherein the controller circuit is configured to determine whether the spot is identifiable based on a disparity in image pixel data in at least a portion of the image.

11. The medical system of claim 10, wherein the controller circuit is configured to determine that the spot is not identifiable when the disparity is below a predetermined threshold.

12. The medical system of claim 1, further comprising an optical fiber operably coupled to the first energy source to direct the treatment energy to the target and to the second energy source to direct the aiming beam to the target.

13. The medical system of claim 12, wherein the optical fiber extends through a treatment-tool channel of the endoscope.

14. The medical system of claim 12, further comprising a sensor operatively coupled to the optical fiber, wherein the controller circuit is configured to determine the selected one of the at least two selectable illumination modes based on illumination light reflected through the optical fiber and detected by the sensor.

15. The medical system of claim 1, wherein the first energy source comprises a treatment laser configured to operate in a cutting mode or a coagulation mode.
